Europäisches Patentamt

European Patent Office

Office européen des brevets

(19)

(11) Publication number: **0 357 315 B1**

(12) EUROPEAN PATENT SPECIFICATION

(45) Date of publication of patent specification: **03.11.93**  (51) Int. Cl.5: **C07D 417/04**, A61K 31/425

(21) Application number: **89308480.6**

(22) Date of filing: **22.08.89**

(54) Hemiphosphate hemihydrate of 2-(1-pentyl-3-guanidino)-4-(2-methyl-4-imidazolyl)thiazole.

(30) Priority: **30.08.88 PCT/US88/03019**

(43) Date of publication of application:
**07.03.90 Bulletin  90/10**

(45) Publication of the grant of the patent:
**03.11.93 Bulletin  93/44**

(84) Designated Contracting States:
**AT BE CH DE ES FR GB GR IT LI LU NL SE**

(56) References cited:
**EP-A- 0 161 841**
**EP-A- 0 178 123**
**EP-A- 0 269 239**
**WO-A-86/02308**
**US-A- 4 560 690**

**JOURNAL OF PHARMACEUTICAL SCIENCES,**
**vol. 66, no. 1, January 1977, Washington, DC**
**(US); S.M. BERGE et al., pp. 1-19&NUM;**

(73) Proprietor: **PFIZER INC.**
**235 East 42nd Street**
**New York, N.Y. 10017(US)**

(72) Inventor: **Appleton, Troy Anthony**
**16, Mistuxet Avenue**
**Mystic, CT(US)**
Inventor: **Smith, Ward Marvin**
**7, Schooner Drive**
**Mystic, CT(US)**

(74) Representative: **Moore, James William, Dr.**
**Pfizer Limited**
**Ramsgate Road**
**Sandwich Kent CT13 9NJ (GB)**

EP 0 357 315 B1

## Description

The present invention is directed to a hemiphosphate hemihydrate salt of 2-(1-pentyl-3-guanidino)-4-(2-methyl-4-imidazolyl)thiazole having advantageous properties.

U.S. Patent 4,560,690, which is herein included by reference, describes 2-(1-pentyl-3-guanidino)-4-(2-methyl-4-imidazolyl)thiazole and analogs as having antiulcer activity. The form of this preferred compound, reported in said patent is the anhydrous dihydrochloride salt, which is noncrystalline, difficult to purify and possesses properties which are generally less suitable for formulation and use as a medicinal agent in man.

International Application No. PCT/US86/02308 reports a further improvement in salts of this preferred antiulcer agent and describes the preparation and properties of the dihydrochloride trihydrate salt.

It has now been found that a yet unreported salt of 2-(1-pentyl-3-guanidino)-4-(2-methyl-4-imidazolyl)-thiazole, namely, the hemiphosphate hemihydrate, offers many advantages especially a high weight percent of active agent.

2-(1-Pentyl-3-guanidino)-4-(2-methyl-4-imidazolyl)thiazole hemiphosphate hemihydrate is prepared by solubilizing the dihydrochloride trihydrate salt (10 mg free base equivalent) per ml in a pH 6.6, 0.2 $\underline{M}$ Phosphate buffer containing acetone in the ratio one part (weight) acetone to four parts (weight) buffer. After standing overnight at room temperature, the desired product is removed by filtration and dried under vacuum at room temperature.

This hemiphosphate hemihydrate salt has all the characteristics desired in a drug to be formulated for human use; it is crystalline, stable, non-hygroscopic and contains a high weight percent of active agent. This latter characteristic is especially important in allowing the formulation of smaller tablets, capsules, etc., for oral administration. This can readily be seen from the following comparison of the various reported forms of 2-(1-pentyl-3-guanidino)-4-(2-methyl-4-imidazolyl)thiazole:

| Form | Molecular Formula | Molecular Wt.(gr/mole) | %Potency[1] |
|---|---|---|---|
| free base | $C_{13}H_{20}N_6S_1$ | 292.4 | 100% |
| dihydrochloride trihydrate | $C_{13}H_{20}N_6S_1$ 2HCl $3H_2O$ | 419.4 | 292.4/419.4 69.7% |
| hemiphosphate hemihydrate | $C_{13}H_{20}N_6S_1$ $1/2H_3PO_4$ $1/2H_2O$ | 350.4 | 292.4/350.4 83.4% |

[1] Potency is defined as the ratio of the molecular weights of the free base to the salt form.

The following example is given by way of illustration:-

EXAMPLE 1

2-(1-Pentyl-3-guanidino)-4-(2-methyl-4-imidazolyl)thiazole Hemiphosphate Hemihydrate Salt

Sufficient 2-(1-pentyl-3-guanidino)-4-(2-methyl-4-imidazolyl)thiazole dihydrochloride trihydrate was added to 100 ml. of a solution consisting, by weight, of 1 part acetone and 4 parts of a 0.2M phosphate buffer to give a drug concentration of 10 mg free base equivalent/ml. The mixture was stirred until all the solids had dissolved and was then allowed to remain undisturbed at room temperature overnight. The precipitated crystals were filtered and dried at room temperature in vacuo for 24 hours.

The DSC data gathered on this material shows it to possess two low temperature endotherms and one high temperature endotherm associated with melting.

The TGA data shows a loss of approximately 3% of the total weight when heated to 170 °C. This weight loss is likely due to water loss, since Karl Fischer readings show 3.2% water on the material.

Samples of the hemiphosphate hemihydrate were submitted for elemental analysis. The amount of carbon, hydrogen and nitrogen are within 3% of the theoretical value whereas phosphorous is slightly higher that the theoretical value. The titratable water is slightly higher than the theoretical water content for a hemihydrate (2.6%). The slightly higher % water could be accounted for by the presence of some surface water on the bulk.

The habit of the hemiphosphate hemihydrate crystals is more rod-like or bladed in character than the needle crystalline habit of the dihydrochloride trihydrate form. Single crystal studies showed that there are 8 molecules per unit cell. The single crystal data confirms that this is the hemiphosphate hemihydrate. X-ray powder diffraction patterns generated for the new form and for the dihydrochloride trihydrate form also verifies that these are two completely different forms.

Samples of the hemiphosphate hemihydrate were stored at 50 ° C. After 55 days, TGA data still shows a weight loss of 2.7% up to 170 ° C and microscopically shows birefringence indicating that the form is physically stable under these conditions.

A tabulation of this information is as follows:

## TABLE 1

### Some Physicochemical Properties of

### 2-(1-pentyl-3-guanidino)-4-(2-methyl-4-imidazolyl)-thiazole hemiphosphate hemihydrate

**1. Thermal Properties**

| | |
|---|---|
| Melting Point °C (Onset of endothermic peak, DSC heating rate = 10°C/min) | 214°C |
| Heat of Fusion (kcal/M) | 4.1 kcal/$\underline{M}$ |
| Percent weight loss (TGA heating rate = 10°C/min) to 170°C | 2.9 (103°-169°C) |

## 2. X-ray and Microscopic Analysis

|  |  |
|---|---|
| Single Crystal | crystalline, consistent with proposed composition |
| Powder Diffraction | crystalline |
| Microscopic Crystallinity | birefringent |

## 3. Elemental Analysis (lot #16362-120-1)

| Element | Theoretical % ($1/2H_3PO_4$ $1/2H_2O$) | Actual % |
|---|---|---|
| C | 44.6 | 44.23 |
| H | 6.5 | 6.48 |
| N | 24.0 | 23.67 |
| P | 4.4 | 4.79 |
| $H_2O$ | 2.6 | 3.2 (KF) |

## 4. Physical Stability after Storage at 50°C (55 Days)

|  |  |
|---|---|
| Percent Weight Loss (TGA) to 170°C. | 2.7% |
| Microscopic Crystallinity | birefringent |

## Claims

### Claims for the following Contracting States : AT BE CH DE FR GB IT LI LU NL SE

1. 2-(1-Pentyl-3-guanidino)-4-(2-methyl-4-imidazolyl)thiazole hemiphosphate hemihydrate.

2. A process for preparing 2-(1-pentyl-3-guanidino)-4-(2-methyl-4-imidazolyl)thiazole hemiphosphate hemihydrate characterized by the steps of (a) combining sufficient 2-(1-pentyl-3-guanidino)-4-(2-methyl-4-imidazolyl)thiazole dihydrochloride trihydrate with a solution comprising, by weight, 1 part acetone and 4 parts of a 0.2M phosphate buffer such that the resulting solution contains 10mg. free base per ml. and (b) filtering and drying the precipitated product.

### Claim for the following Contracting States : ES GR

1. A process for preparing 2-(1-pentyl-3-guanidino)-4-(2-methyl-4-imidazolyl)thiazole hemiphosphate hemihydrate characterized by the steps of (a) combining sufficient 2-(1-pentyl-3-guanidino)-4-(2-methyl-4-imidazolyl)thiazole dihydrochloride trihydrate with a solution comprising, by weight, 1 part acetone and 4 parts of a 0.2M phosphate buffer such that the resulting solution contains 10mg. free base per ml. and (b) filtering and drying the precipitated product.

**Patentansprüche**

**Patentansprüche für folgende Vertragsstaaten : AT BE CH DE FR GB IT LI LU NL SE**

1. 2-(1-Pentyl-3-guanidino)-4-(2-methyl-4-imidazolyl)-thiazol-Hemiphosphat-Hemihydrat.

2. Verfahren zur Herstellung von 2-(1-Pentyl-3-guanidino)-4-(2-methyl-4-imidazolyl)-thiazol-Hemiphosphat-Hemihydrat, gekennzeichnet durch die Schritte (a) Vereinigen von ausreichend 2-(1-Pentyl-3-guanidi-no)-4-(2-methyl-4-imidazolyl)-thiazol-Dihydrochloridtrihydrat mit einer Lösung, umfassend, aufs Gewicht bezogen, 1 Teil Aceton und 4 Teile eines 0,2 M Phosphatpuffers, so daß die erhaltene Lösung 10 mg freie Base pro ml enthält und (b) Filtrieren und Trocknen des ausgefallenen Produkts.

**Patentanspruch für Vertragsstaaten : ES GR**

1. Verfahren zur Herstellung von 2-(1-Pentyl-3-guanidino)-4-(2-methyl-4-imidazolyl)-thiazol-Hemiphosphat-Hemihydrat, gekennzeichnet durch die Schritte (a) Vereinigen von ausreichend 2-(1-Pentyl-3-guanidi-no)-4-(2-methyl-4-imidazolyl)-thiazol-Dihydrochloridtrihydrat mit einer Lösung, umfassend, aufs Gewicht bezogen, 1 Teil Aceton und 4 Teile eines 0,2 M Phosphatpuffers, so daß die erhaltene Lösung 10 mg freie Base pro ml enthält und (b) Filtrieren und Trocknen des ausgefallenen Produkts.

**Revendications**

**Revendications pour les Etats contractants suivants : AT, BE, CH, DE, FR, GB, IT, LI, LU, NL, SE**

1. Hémihydrate d'hémiphosphate de 2-(1-pentyl-3-guanidino)-4-(2-méthyl-4-imidazolyl)thiazole.

2. Procédé de préparation d'hémihydrate d'hémiphosphate de 2-(1-pentyl-3-guanidino)-4-(2-méthyl-4-imidazolyl)thiazole, caractérisé par les étapes (a) de mélange, à une solution comprenant, en poids, 1 partie d'acétone et 4 parties d'un tampon au phosphate 0,2 M, d'une quantité de trihydrate de dichlorhydrate de 2-(1-pentyl-3-guanidino)-4-(2-méthyl-4-imidazolyl)thiazole suffisante pour que la solution résultante contienne 10 mg de base libre par ml, et (b) de filtration et de séchage du produit précipite.

**Revendication pour les Etats contractants suivants : ES, GR**

1. Procédé de préparation d'hémihydrate d'hémiphosphate de 2-(1-pentyl-3-guanidino)-4-(2-méthyl-4-imidazolyl)thiazole, caractérisé par les étapes (a) de mélange, à une solution comprenant, en poids, 1 partie d'acétone et 4 parties d'un tampon au phosphate 0,2 M, d'une quantité de trihydrate de dichlorhydrate de 2-(1-pentyl-3-guanidino)-4-(2-méthyl-4-imidazolyl)thiazole suffisante pour que la solution résultante contienne 10 mg de base libre par ml, et (b) de filtration et de séchage du produit précipité.